# EUROPEAN PATENT SPECIFICATION

(11) **EP 3 678 737 B1**
(45) Date of publication and mention of the grant of the patent: **24.08.2022**
(21) Application number: 18769435.1
(22) Date of filing: 05.09.2018
(51) Int. Cl.: A61N 5/06

(54) **SELF-ADHESIVE PHOTOTHERAPY TREATMENT DEVICE**
SELBSTKLEBENDE PHOTOTHERAPIEBEHANDLUNGSVORRICHTUNG
DISPOSITIF DE TRAITEMENT PAR PHOTOTHÉRAPIE AUTO-ADHÉSIF

(30) Priority: 05.09.2017 GB 201714230
(43) Date of publication of application: 15.07.2020
(73) Proprietor: Ambicare Health Limited, Livingston EH54 7GA (GB)
(72) Inventor: MACGILP, Neil Archibald, Livingston EH54 7GA (GB); LOW, Graeme, Livingston EH54 7GA (GB); PETERSEN, Ronald John, Livingston EH54 7GA (GB)
(74) Representative: HGF
(86) International application number: PCT/GB2018/052511
(87) International publication number: WO 2019/048849

(56) References cited:
- US-A1- 2010 121 252
- US-A1- 2013 178 919
- US-A1- 2016 236 007

## Description

### FIELD OF THE INVENTION

The present invention relates to a phototherapy treatment device for use in therapeutic treatment of a variety of skin conditions such as acne and psoriasis, non-melanoma skin cancer and wound healing as well as cosmetic treatments including wrinkle reduction.

### BACKGROUND OF THE INVENTION

Reference is made to documents US2013/0178919 A1 and US2016/0236007 A1. The treatment of common skin conditions such as acne and psoriasis has to date mainly been centred in Hospitals and Dermatology Centres. Users tend to have to use eye protection while phototherapy is applied to the skin area. This obviously limits access to treatment to those mainly living in urban areas and to those who are able to attend outpatient treatment centres. This therefore vastly reduces the ability of patents who are unable to attend patient treatment centres to receive the required treatment. Moreover, this can also cause severe disruption to people required to attend their workplace by having to obtain regular absence from their work. Therefore, using previous treatments, the treatment experience is therefore highly inconvenient for users.

It is therefore an object of at least one aspect of the present invention to obviate or mitigate at least one or more of the aforementioned problems.

It is a further object of the present invention to improve treating common skin conditions such as acne and psoriasis.

It is the further object of the present invention to provide a phototherapy treatment device which comprises LED light sources, a battery and control electronics which allows ambulatory treatment to be applied to a patient.

### STATEMENT OF INVENTION

The invention is defined in the appended set of claims.

According to the first aspect of the present invention there is provided a self-adhesive ambulatory phototherapy treatment device for use in therapeutic and/or cosmetic treatments wherein the device comprises:
a double-sided self-adhesive layer which is capable of attaching itself to a patient to be treated, the self-adhesive layer being substantially transparent to wavelength of light used for the phototherapy treatment; and
wherein the self-adhesive ambulatory phototherapy treatment device includes a treatment head comprising of a light source, a battery and control electronics.

The device of the present invention may therefore be used in the phototherapy treatment of a variety of common skin conditions such as acne, psoriasis, non-melanoma skin cancer and wound healing. The device of the present invention may also be used in cosmetic treatment of the skin, such as in wrinkle reduction.

Typically, the light source is located on the side of the treatment head that is facing the skin during use, or skin-facing side, such that the light emitted by the light source is directed at the skin to be treated.

The double-sided self-adhesive layer may be at least partially located over the light source. The double-sided self-adhesive layer may be located directly over the light source. Accordingly, light emitted by the light source may be at least partially transmitted through the double-sided self-adhesive layer.

Therefore, the double-sided self-adhesive layer is substantially transparent to the light used for the phototherapy treatment.

The double-sided self-adhesive layer may be replaceable and may be constructed from common double-sided tape. The double-sided self-adhesive layer may comprise a medical grade adhesive such that the device may be adhered or attached to the skin of a patient and be retained thereon during the treatment and be readily removed from the skin of the patient once the treatment is complete. Preferably, the adhesive does not adversely affect the skin of the patient when the device is adhered to the skin of the patient. Preferably, the adhesive does not adversely affect the skin of the patient when the adhered device is removed from the skin of the patient.

Typical devices known in the art require the patient to hold the device in place during the treatment process. Accordingly, the arm of the patient that is being used to hold the device in place may tire during the treatment and may lead to the patient removing the device before the treatment is completed. In contrast, the device of the present aspect is adhered to the skin of the patient, thereby leaving the hands of the patient free and making it more likely that the patient will leave the device in place for the full length of the treatment.

A double-sided self-adhesive layer may have a transmission of greater than about 95% of the wavelength of light used in phototherapy treatment.

In preferred embodiments, the light transmission may be greater than about 97% for the utilised wavelength.

The light source may be a plurality of LED light sources, a single OLED sheet of light emitting material or a single high powered LED with a diffuser.

The light source may be a plurality of light sources such as LEDs.

The light source may be an OLED where the light is emitted from a surface rather than a point or a series of points.

The light source may emit a wavelength of light in the region of about 300 nm - 900 nm.

Furthermore, the emitted light may comprise a combination of more than one wavelength of light such as two different wavelengths of light. The different wavelengths of light may come from the blue such as the UV region and/or red light such as from the IR wavelength region. In particular embodiments the wavelength of light used may be about 400 nm - 450 nm (e.g. about 415 nm) and/or about 600 nm - 700 nm (e.g. about 640 nm). The blue light (400 nm - 450 nm) has been found to be useful in treating acne bacteria and the red light (600 nm - 700 nm) to reduce inflammation, to treat non-melanoma skin cancer and to treat wrinkles. Blue light may therefore be used on its own. Red light may be used on its own. Alternatively, both red light and blue light may be used in combination.

A specific feature of the present invention is that the device is ambulatory (i.e. it can be used whilst walking) and is therefore light weight enough for a patient to wear and carry around conveniently during their day-to-day activities such as at the workplace or conduct activities around the household. The device is not hard wired to a control device or power supply. Rather, the battery and control electronics are contained within the treatment head, resulting in a device that is more portable and more convenient to use than devices connected to external power supplies and /or controls. Furthermore, the elimination of the requirement for external controls and power supply has resulted in significant savings in cost for the device.

In particular embodiments, the phototherapy device of the present invention may therefore have a ratio of device weight (gm) to the skin contact area (cm²) in the range of about 4.0 gm/cm² to 1.0 gm/cm². The phototherapy device may have a ratio of device weight to skin contact area in the range of about 3.0 gm/cm² to 2.0 gm/cm². In particularly preferred embodiments, the ratio of device weight (gm) to the skin contact area (cm²) is ideally below about 2.5 gm/cm².

The phototherapy treatment device may also preferentially comprise a flexible cuff. The flexible cuff may extend around a treatment head of the device to contain the light and prevent light leakage during application of light to a skin area. This also has a safety function of reducing any potential negative consequences for eye safety to a user and other surrounding persons.

In preferred embodiments, the device may be flexible and comprise a flexible treatment head area. This may allow the device to conform to a variety of curved and/or contoured shaped skin surfaces such as a leg, torso, or facial area, for example.

The LED light sources in preferred embodiments may be organic LEDs (i.e. OLEDs).

The LED light sources may extend around the treatment head area providing a uniform and constant light intensity meaning that an even phototherapy treatment is applied to a patient's skin area. The LED light sources may be arranged in a uniform manner across the skin-facing side of the treatment head. For example, the LED light sources may be arranged in a grid pattern, or otherwise equally spaced across the skin-facing side of the treatment head.

Preferably, the LED light sources are very efficient, and therefore use a lower amount of electrical power to emit a given intensity of light than less efficient LED light sources. For example, the LED light sources may be more than 30% efficient, more than 35% efficient, more than 40% efficient, more than 45% efficient or more than 50% efficient. Accordingly, more than 30%, 35%, 40%, 45%, or 50% of the electrical energy received by the LED light sources may be converted into light, with the remainder being lost as heat, for example. The device may comprise a cooling element to dissipate heat generated by the light source. The device may comprise a heat sink to dissipate heat generated by the light source. For example, in embodiments comprising a plurality of LED light sources, the heat sink may dissipate heat generated by one or more of the plurality of LED light sources.

The device may comprise a vent that allows heat generated by the light source to escape the device. In embodiments comprising a heat sink, the heat dissipated from the heat sink may escape the device via the vent.

Due to the high efficiency of the LED light sources, a smaller battery power supply may be used in the device, thereby reducing the weight of the device for a given area of skin to be treated.

The device may provide a predetermined flux of light to the skin of a user for a predetermined amount of time when the device is activated. The device may provide a predetermined flux of light to the skin of a user for 10 minutes, 15 minutes, 20 minutes, 25 minutes or 30 minutes or more. The device may provide a predetermined flux of light for 15 minutes or 20 minutes. The device may provide a predetermined flux of light for 20 minutes.

The control electronics may be configured to activate the light source when the device is activated. The control electronics may be configured to determine the period of time for which the light source will emit light. Accordingly, the device may not comprise controls that would allow the user to adjust the period of time for which the light source will emit light. As a result, the device may be simplified and be more light-weight than devices that comprise controls that allow the user to adjust the period of time for which the light source will emit light.

The device may comprise an activation button. The device may comprise a wireless receiver that is configured to activate the device when it receives a suitable signal from a remote source.

The device may provide about 1 - 10 mW/cm² of light to the skin of the patient. The device may provide about 3 - 7 mW/cm² of light to the skin of the patient. The device may provide about 4 - 6 mW/cm² of light to the skin of the patient. The device may provide 1, 2, 3, 4, 5, 6, 7, 8, 9, or 10 mW/cm² of light to the skin of the patient. For example, the device may provide 3, 4, or 5 mW/cm² of light to the skin of the patient.

It is a preferred feature of the present invention in that the device is lightweight and that the battery is not too heavy thereby allowing the device to be ambulatory. The preferred battery source for the invention is a lithium ion type battery which can be recharged. The control electronics are also specifically designed to be as lightweight as possible. The battery may be recharged without the device having to be dismantled to access the battery. Accordingly, the device may comprise a port that is configured to receive a power adapter to thereby recharge the battery of the device. Alternatively, the battery may be configured to be recharged inductively. Therefore, the device may be placed on a dock or similar to recharge the battery without the need to plug the device into a power lead or similar.

The device may be configured to be linked wirelessly with a remote device. The remote device may be a smart watch, smart phone, tablet or computer, for example. Accordingly, the user may be provided with increased data capture, diagnostics and usage patterns thereby enabling increased treatment compliance and treatment efficacy.

The device may be used in combination with a therapeutic agent which can be applied to the skin and activated or enabled by light i.e. photodynamic therapy (PDT). For example, the device may comprise a light source that emits red light and may be used in the treatment of non-melanoma skin cancer. Prior to the treatment of the skin using the device, a therapeutic agent may be applied to the skin. Accordingly, once the therapeutic agent has been absorbed into the skin, an active form of the therapeutic agent may be created when irradiated with red light from the device. Non-limiting examples of a suitable therapeutic agents include aminolevulinic acid (ALA), or methyl aminolevulinate (such as the product sold under the brand name Metvix^{™}).

In another example, the device may comprise a light source that emits red light and may be used in the treatment of wrinkles. Prior to the treatment of the skin using the device a therapeutic agent may be applied to the skin. Accordingly, once the therapeutic agent has been absorbed into the skin, an active form of the therapeutic agent may be created when irradiated with red light from the device. A non-limiting example of a suitable therapeutic agent is aminolevulinic acid (ALA).

The device may be used in conjunction with a therapeutic agent which is activated at the appropriate time point by exposure to light of a specific wavelength for beneficial impact on the skin being treated.

The method may comprise the steps of providing a device according to the first aspect, attaching the device to the skin to be treated, activating the device to thereby expose the skin to the light emitted by the light source of the device, waiting for a predetermined period of time, and removing the device from the skin.

Accordingly, the method may allow for treatment of conditions of the skin. The method may allow treatment of skin conditions such as acne, eczema, psoriasis, inflammation, wound healing. The method may allow treatment of cosmetic conditions such as wrinkles.

The device may automatically deactivate after the predetermined period of time.

According to a third aspect of the present invention there is provided a use of the device of the first aspect to treat skin conditions. The skin condition may be selected from the group acne, eczema, psoriasis, inflammation or wounds. In embodiments using the device to treat acne, the device may comprise light sources that emit blue light. In embodiments using the device to treat inflammation, the device may comprise light sources that emit red light.

### BRIEF DESCRIPTION OF THE FIGURES

Embodiments of the present invention will now be described, by way of example only, with reference to the accompanying drawings in which:
Figure 1 is a perspective view of a phototherapy treatment device according to present invention;
Figure 2 is a side view of the phototherapy treatment device shown in Figure 1;
Figure 3 is a top view of a device according to an embodiment;
Figure 4 is a side view of a device according to an embodiment;
Figure 5 is a bottom view of a device according to an embodiment;
Figure 6 is a front view of a device according to an embodiment;
Figure 7 is a top view of a device according to an embodiment;
Figure 8 is a bottom view of a device according to an embodiment;
Figure 9 is a bottom view of a device according to an embodiment; and
Figure 10 is a view of a double-sided self-adhesive layer suitable for the device an embodiment.

### DETAILED DESCRIPTION

Generally speaking, the present invention resides in the provision of an improved phototherapy treatment device for the ambulatory treatment of a patient for the therapeutic and/or cosmetic treatment of a variety of skin conditions such as acne and psoriasis, non-melanoma skin cancer and wound healing as well as cosmetic treatments including wrinkle reduction.

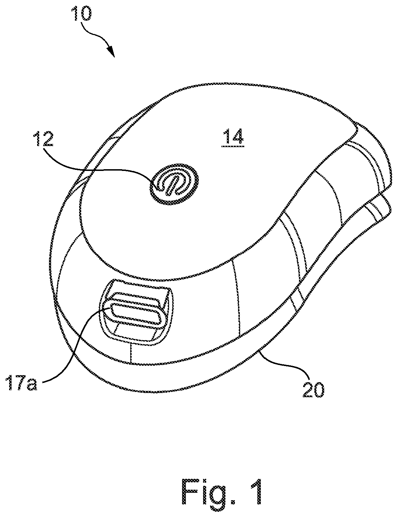

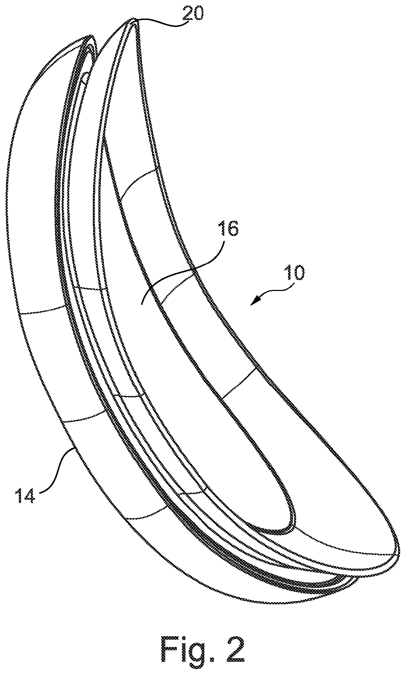

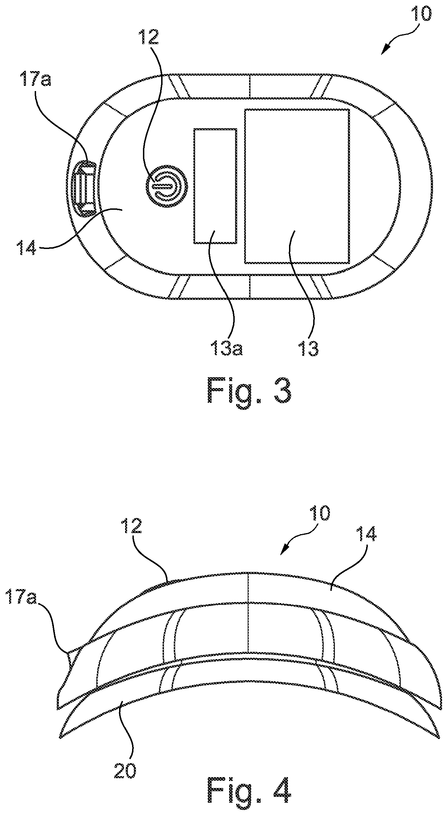

Figures 1 to 6 show a phototherapy treatment device according to the present invention generally designated 10. The phototherapy treatment device 10 comprises an activation button 12 which can be used to activate the device by a user when treatment is required.

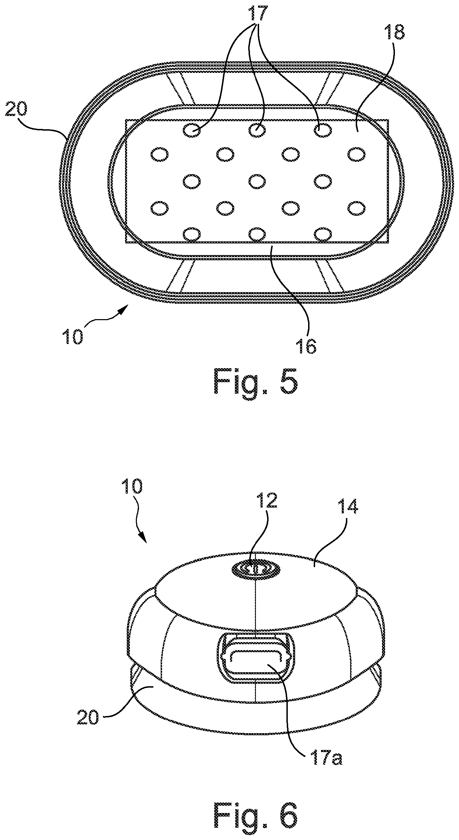

The phototherapy treatment device has an upper surface 14 and a bottom surface which form a treatment head 16 through which treatment can be applied. The treatment head 16 comprises a plurality of LED light sources (examples shown as 17 in Figure 5), which can be used to emit a wavelength of light from anywhere in the region of about 300 - 900 nm. Preferably, the emitted light has a wavelength of between 415 and 640 nm. The LED light sources 17 are uniformly located over the treatment head area 16 and therefore apply a constant and even intensity of light to an area of skin.

The phototherapy treatment device 10 comprises a battery 13 and control electronics 13a. The battery 13 provides power to the control electronics 13a and the plurality of LED light sources 17, and the control electronics control the intensity of light emitted by the plurality of LED light sources 17 and the period of time for which the plurality of LED light sources 17 emit light.

The phototherapy treatment device 10 comprises a recharge port 17a to allow the battery 13 to be recharged.

Alternatively the light emitted comes from the blue such as the UV region and/or red light such as from the IR wavelength region. In particular embodiments the wavelength of light used is about 400 nm - 450 nm (e.g. about 415 nm) and/or about 600 nm - 700 nm (e.g. about 640 nm). The blue light has been found to be useful in treating acne bacteria and the red light has been found to be useful to reduce inflammation. The emitted light may comprise a combination of more than one wavelength of light such as two different wavelengths of light

On the front surface of the treatment head 16 there is a double-sided self-adhesive layer 18 which can be used to attach the phototherapy treatment device 10 to an area of a patient such as a leg, torso or facial area. The double-sided self-adhesive layer 18 is replaceable and can therefore be replaced after it has been used a number of times by a user. In addition, the double-sided self-adhesive layer 18 is substantially transparent to the light being emitted by the LED light sources 17. The double-sided self-adhesive layer 18 is a double coated medical tape (Item 1522 from 3M^{™}) comprising polyethylene tape coated in a medical grade acrylate adhesive. The adhesive on the skin facing side is covered with a release paper, which is removed before use.

Preferably, the double-sided self-adhesive layer 18 has a transmission of greater than about 95% of the emitted light from the LED light sources 17.

The phototherapy treatment device of the present invention is manufactured to be as lightweight as possible and preferably has a weight of less than about 2.5 gm per cm² of the treatment surface. The battery powering the phototherapy treatment device and the controlling electronics are therefore as lightweight as possible meaning that the device of the present invention is ambulatory and can be utilised by a user at home and be worn during their day-to-day business such as being used at their workplace or when they are conducting household activities.

The phototherapy treatment device 10 may also comprise a flexible cuff 20 around the treatment head 16 which can be used to contain the light being emitted by the LED light sources 17. This also prevents any damage to the eyes of a user.

The phototherapy treatment device 10 and the treatment head 16 are flexible which allows the treatment head 16 to adapt and conform to the contours of a user surface such as around a leg, torso or facial area.

In an alternative embodiment, the LED light sources are organic LEDs.

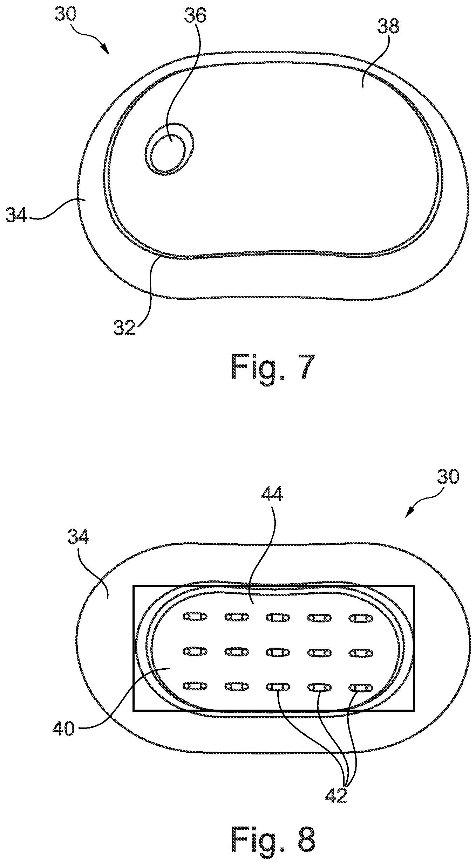

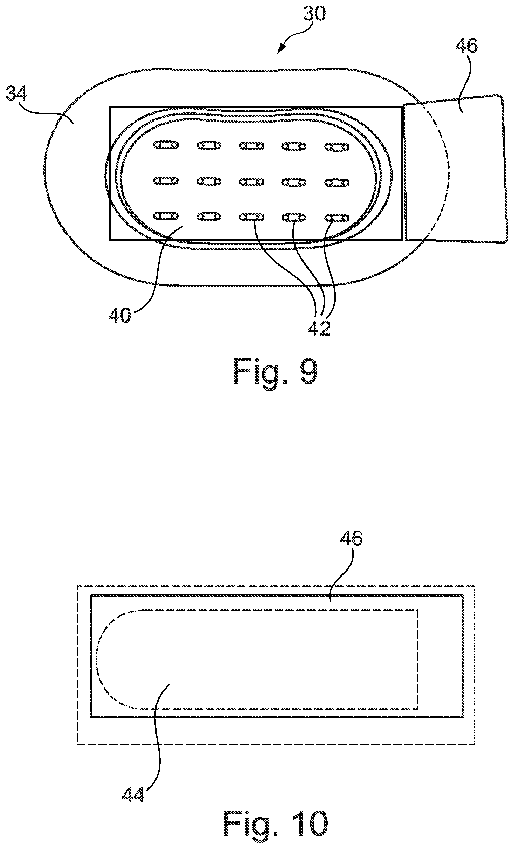

In a further example, shown in Figures 7-10, a device 30 for the treatment of acne comprises a main body 32, and a flexible cuff 34 running around the periphery of the main body 32. The main body 32 comprises an activation button 36 on a top side 38. The main body 32 also comprises a skin facing side 40 and a plurality of LED light sources (examples shown as 42 in Figures 8 and 9) arranged in a grid pattern across the skin facing side 40 of the main body 32 (see Figure 8, for example). The plurality of LED light sources 42 are configured to emit light at a wavelength of 415 nm +/- 15 nm (blue light).

The main body 32 further comprises an adhesive layer 44 provided over the plurality of LED light sources 42. Within the main body 32 is housed a battery and control electronics (not shown).

Before use, the adhesive layer 44 is protected by a cover sheet 46.

When the device 30 is to be used to treat acne, the user removes the cover sheet 46 from the adhesive layer 44 and applies the device 30 to the area of skin to be treated such that the skin facing surface of the main body is directly adjacent to the skin and the adhesive contacts the skin.

The user then presses the activation button 36. The control electronics activate the plurality of LED light sources 42, which then emit light onto the skin at a flux of 5 mW/cm² for 20 minutes. When the 20 minute period expires, the control electronics deactivate the plurality of LED light sources and the device 30 may be removed from the skin by the user.

It will be appreciated by the person skilled in the art that the device may be used to treat any skin condition that can be treated by exposure to blue light and the period of time that the plurality of LED light sources are activated can be varied as required for a given specific treatment.

In alternative embodiments, the device comprises a plurality of LED light sources configured to emit light at a wavelength of 640 nm +/- 10 nm (red light), and the device may be used to treat any skin condition that can be treated by exposure to red light, such as inflammation, or to be used in wrinkle reduction. Furthermore, a device comprising red LED light sources may be used to treat non-melanoma skin cancer or wrinkles in combination with the application of a suitable therapeutic agent such as ALA prior to the attachment of the device to the area of skin to be treated.

While in its simplest form, the device once charged, and switched on, is self contained and provides the designed level of irradiance, for a predetermined time period and thereafter switches itself off, further external control and monitoring through Bluetooth type connectivity to a smart watch, phone, tablet or computer is an option, allowing users, for example to track their usage over time for maximum therapeutic effects..

Whilst specific embodiments of the present invention have been described above, it will be appreciated that departures from the described embodiments may still fall within the scope of the present invention. For example, any suitable type of light emitting device may be used to provide the therapeutic treatment. Moreover, any form of battery source and control electronics may be used.

## Claims

1. A self-adhesive ambulatory phototherapy treatment device for therapeutic and/or cosmetic treatments, wherein the device comprises:
a treatment head comprising at least one LED light source, a battery and control electronics, a double-sided self-adhesive layer which is capable of attaching itself to a patient to be treated, the self-adhesive layer located over the at least one LED source and being substantially transparent to wavelength of light used for the phototherapy treatment;
and a flexible cuff extending around the treatment head to contain the light and prevent light leakage during application of light to a patient's skin area.

2. A self-adhesive ambulatory phototherapy treatment device according to any preceding claim, wherein the double-sided self-adhesive layer is replaceable and is constructed from double-sided tape.

3. A self-adhesive ambulatory phototherapy treatment device according to any preceding claim, wherein the double-sided self-adhesive layer has a light transmission of greater than about 95% of the wavelength of light used in phototherapy treatment.

4. A self-adhesive ambulatory phototherapy treatment device according to any preceding claim, wherein the double-sided self-adhesive layer has a light transmission of greater than about 97% of the wavelength of light used in phototherapy treatment.

5. A self-adhesive ambulatory phototherapy treatment device according to any preceding claim, wherein the wavelength of light used is in the region of about 300-900nm.

6. A self-adhesive ambulatory phototherapy treatment device according to any preceding claim, wherein the wavelength of light used is in the region of between 415 and 640 nm.

7. A self-adhesive ambulatory phototherapy treatment device according to any preceding claim, wherein the device is used in conjunction with an additional therapeutic agent which is activated at the appropriate time point by exposure to light of a specific wavelength for beneficial impact on the skin being treated.

8. A self-adhesive ambulatory phototherapy treatment device according to any preceding claim, wherein the phototherapy device of the present invention has a ratio of device weight (gm) to the skin contact area (cm²) in the range of about 4.0 gm/cm² to 1.0 gm/cm².

9. A self-adhesive ambulatory phototherapy treatment device according to any preceding claim, wherein the phototherapy device of the present invention has a ratio of device weight (gm) to the skin contact area (cm²) in the range of 2.5 gm/cm² or less.

10. A self-adhesive ambulatory phototherapy treatment device according to any preceding claim, wherein the device is flexible and comprises a flexible treatment head area which allows the device to conform to a variety of curved and/or contoured shaped skin surfaces.

11. A self-adhesive ambulatory phototherapy treatment device according to any preceding claim, wherein the at least one LED light source comprises organic light-emitting diodes.

12. A self-adhesive ambulatory phototherapy treatment device according to any preceding claim, wherein the at least one LED source comprises a plurality of LED light sources which extend around the treatment head area providing a uniform and constant light intensity so as to apply an even phototherapy treatment the patient's skin area.

13. A self-adhesive ambulatory phototherapy treatment device according to any preceding claim, wherein the battery source for the invention is a rechargeable lithium ion type

14. A self-adhesive ambulatory phototherapy device according to any preceding claim, wherein the device is configured to be linked wirelessly with a remote device.

## Patentansprüche

1. Selbstklebende ambulante Phototherapiebehandlungsvorrichtung für therapeutische und/oder kosmetische Behandlungen, wobei die Vorrichtung Folgendes umfasst:
einen Behandlungskopf, der mindestens eine LED-Lichtquelle, eine Batterie und eine Steuerelektronik umfasst,
eine doppelseitige selbstklebende Schicht, die dazu in der Lage ist, sich selbst an einem zu behandelnden Patienten anzubringen, wobei sich die selbstklebende Schicht über der mindestens einen LED-Quelle befindet und für die Wellenlänge des Lichts, das für die Phototherapiebehandlung verwendet wird, im Wesentlichen transparent ist;
und
eine flexible Manschette, die sich um den Behandlungskopf erstreckt, um das Licht einzudämmen und ein Austreten von Licht während der Anwendung von Licht auf eine Hautfläche des Patienten zu verhindern.

2. Selbstklebende ambulante Phototherapiebehandlungsvorrichtung nach einem vorhergehenden Anspruch, wobei die doppelseitige selbstklebende Schicht austauschbar ist und aus doppelseitigem Klebeband aufgebaut ist.

3. Selbstklebende ambulante Phototherapiebehandlungsvorrichtung nach einem vorhergehenden Anspruch, wobei die doppelseitige selbstklebende Schicht eine Lichtdurchlässigkeit von mehr als etwa 95 % der bei der Phototherapiebehandlung verwendeten Lichtwellenlänge aufweist.

4. Selbstklebende ambulante Phototherapiebehandlungsvorrichtung nach einem vorhergehenden Anspruch, wobei die doppelseitige selbstklebende Schicht eine Lichtdurchlässigkeit von mehr als etwa 97 % der bei der Phototherapiebehandlung verwendeten Lichtwellenlänge aufweist.

5. Selbstklebende ambulante Phototherapiebehandlungsvorrichtung nach einem vorhergehenden Anspruch, wobei die verwendete Lichtwellenlänge in der Region von etwa 300-900 nm liegt.

6. Selbstklebende ambulante Phototherapiebehandlungsvorrichtung nach einem vorhergehenden Anspruch, wobei die verwendete Lichtwellenlänge in der Region zwischen 415 und 640 nm liegt.

7. Selbstklebende ambulante Phototherapiebehandlungsvorrichtung nach einem vorhergehenden Anspruch, wobei die Vorrichtung in Verbindung mit einem zusätzlichen therapeutischen Mittel verwendet wird, das zum geeigneten Zeitpunkt aktiviert wird, indem es Licht einer spezifischen Wellenlänge ausgesetzt wird, um eine vorteilhafte Wirkung auf die behandelte Haut auszuüben.

8. Selbstklebende ambulante Phototherapiebehandlungsvorrichtung nach einem vorhergehenden Anspruch, wobei die Phototherapievorrichtung der vorliegenden Erfindung ein Verhältnis von Vorrichtungsgewicht (gm) zu Hautkontaktfläche (cm²) im Bereich von etwa 4,0 g/cm² bis 1,0 g/cm² aufweist.

9. Selbstklebende ambulante Phototherapiebehandlungsvorrichtung nach einem vorhergehenden Anspruch, wobei die Phototherapievorrichtung der vorliegenden Erfindung ein Verhältnis von Vorrichtungsgewicht (gm) zu Hautkontaktfläche (cm²) im Bereich von 2,5 g/cm² oder weniger aufweist.

10. Selbstklebende ambulante Phototherapiebehandlungsvorrichtung nach einem vorhergehenden Anspruch, wobei die Vorrichtung flexibel ist und eine flexible Behandlungskopffläche umfasst, der es der Vorrichtung ermöglicht, sich an eine Vielfalt von gekrümmten und/oder konturierten Hautoberflächen anzupassen.

11. Selbstklebende ambulante Phototherapiebehandlungsvorrichtung nach einem vorhergehenden Anspruch, wobei die mindestens eine LED-Lichtquelle organische Leuchtdioden umfasst.

12. Selbstklebende ambulante Phototherapiebehandlungsvorrichtung nach einem vorhergehenden Anspruch, wobei die mindestens eine LED-Quelle eine Vielzahl von LED-Lichtquellen umfasst, die sich um die Behandlungskopffläche erstreckt und eine gleichmäßige und konstante Lichtintensität zum Anwenden einer gleichmäßigen Phototherapiebehandlung der Hautfläche des Patienten bereitstellen.

13. Selbsthaftende ambulante Phototherapiebehandlungsvorrichtung nach einem vorhergehenden Anspruch, wobei die Batteriequelle für die Erfindung ein wiederaufladbarer Lithiumionen-Typ ist.

14. Selbsthaftende ambulante Phototherapievorrichtung nach einem vorhergehenden Anspruch, wobei die Vorrichtung dazu konfiguriert ist, drahtlos mit einer entfernten Vorrichtung verbunden zu werden.

## Revendications

1. Dispositif de traitement photothérapeutique ambulatoire auto-adhésif destiné à des traitements thérapeutiques et/ou cosmétiques, dans lequel le dispositif comprend :
une tête de traitement comprenant au moins une source lumineuse LED, une batterie et des composants électroniques de commande,
une couche autocollante double face qui est en mesure de se fixer sur un patient à traiter, la couche auto-adhésive située au-dessus de l'au moins une source LED et étant sensiblement transparente à la longueur d'onde de lumière utilisée pour le traitement de photothérapie ;
et
un manchon flexible s'étendant autour de la tête de traitement pour contenir la lumière et empêcher la fuite de lumière lors de l'application de lumière sur la zone cutanée d'un patient.

2. Dispositif de traitement photothérapeutique ambulatoire auto-adhésif selon l'une quelconque des revendications précédentes, dans lequel la couche auto-adhésive double face est remplaçable et est constituée d'un ruban adhésif double face.

3. Dispositif de traitement photothérapeutique ambulatoire auto-adhésif selon l'une quelconque des revendications précédentes, dans lequel la couche auto-adhésive double face a une transmission lumineuse supérieure à environ 95 % de la longueur d'onde de lumière utilisée dans le traitement photothérapeutique.

4. Dispositif de traitement photothérapeutique ambulatoire auto-adhésif selon l'une quelconque des revendications précédentes, dans lequel la couche auto-adhésive double face a une transmission lumineuse supérieure à environ 97 % de la longueur d'onde de lumière utilisée dans le traitement photothérapeutique.

5. Dispositif de traitement photothérapeutique ambulatoire auto-adhésif selon l'une quelconque des revendications précédentes, dans lequel la longueur d'onde de lumière utilisée se situe dans la région d'environ 300 à 900 nm.

6. Dispositif de traitement photothérapeutique ambulatoire auto-adhésif selon l'une quelconque des revendications précédentes, dans lequel la longueur d'onde de lumière utilisée se situe dans la région comprise entre 415 et 640 nm.

7. Dispositif de traitement photothérapeutique ambulatoire auto-adhésif selon l'une quelconque des revendications précédentes, dans lequel le dispositif est utilisé en conjonction avec un agent thérapeutique supplémentaire qui est activé au moment approprié par exposition à la lumière d'une longueur d'onde spécifique pour un impact bénéfique sur la peau en cours de traitement.

8. Dispositif de traitement photothérapeutique ambulatoire auto-adhésif selon l'une quelconque des revendications précédentes, dans lequel le dispositif de photothérapie de la présente invention a un rapport du poids du dispositif (gm) à la surface de contact cutané (cm²) dans la plage d'environ 4,0 g/cm² à 1,0 g/cm².

9. Dispositif de traitement photothérapeutique ambulatoire auto-adhésif selon l'une quelconque des revendications précédentes, dans lequel le dispositif de photothérapie de la présente invention a un rapport du poids du dispositif (gm) à la surface de contact cutané (cm²) dans la plage de 2,5 g/cm² ou moins.

10. Dispositif de traitement photothérapeutique ambulatoire auto-adhésif selon l'une quelconque des revendications précédentes, dans lequel le dispositif est flexible et comprend une zone de tête de traitement flexible qui permet au dispositif de se conformer à une variété de surfaces cutanées de forme incurvée et/ou profilée.

11. Dispositif de traitement photothérapeutique ambulatoire auto-adhésif selon l'une quelconque des revendications précédentes, dans lequel l'au moins une source de lumière LED comprend des diodes électroluminescentes organiques.

12. Dispositif de traitement photothérapeutique ambulatoire auto-adhésif selon l'une quelconque des revendications précédentes, dans lequel l'au moins une source de LED comprend une pluralité de sources de lumière LED qui s'étendent autour de la zone de la tête de traitement fournissant une intensité lumineuse uniforme et constante afin d'appliquer un traitement photothérapeutique uniforme sur la zone cutanée du patient.

13. Dispositif de traitement photothérapeutique ambulatoire auto-adhésif selon l'une quelconque des revendications précédentes, dans lequel la source de batterie pour l'invention est de type lithium-ion rechargeable.

14. Dispositif de photothérapie ambulatoire auto-adhésif selon l'une quelconque des revendications précédentes, dans lequel le dispositif est configuré pour être relié sans fil à un dispositif distant.
